# EUROPÄISCHE PATENTSCHRIFT

(11) **EP 0 873 763 B1**
(45) Veröffentlichungstag und Bekanntmachung des Hinweises auf die Patenterteilung: **13.03.2002**
(21) Anmeldenummer: 98106771.3
(22) Anmeldetag: 09.04.1998
(51) Int. Cl.: A61M 39/00

(54) **Katheterventil**
Catheter valve
Soupape pour cathéter

(30) Priorität: 24.04.1997 DE 29707410 U
(43) Veröffentlichungstag der Anmeldung: 28.10.1998
(73) Patentinhaber: CareMed Medical Produkte Aktiengesellschaft, 01109 Dresden (DE)
(72) Erfinder: Pfleiderer, Klaus, 60433 Frankfurt/Main (DE); Heise, Peter, 34277 Fuldabrück (DE)
(74) Vertreter: Pätzelt, Peter, Dipl.-Ing.

(56) Entgegenhaltungen:
- EP-A- 0 389 402
- WO-A-93/24173
- DE-C- 532 357
- DE-U- 29 700 657
- US-A- 5 228 646

## Beschreibung

Die Erfindung betrifft ein Katheterventil zum Verschluss transurethraler und suprapubischer Katheter. Es kann auch als Entleerungsventil für Urinbeutel eingesetzt werden. Es ist aber auch zur Ableitung anderer Körperflüssigkeiten, die über einen Katheter intermittierend oder permanent abgeleitet werden sollen, geeignet. Bei Schlauchanschluss und permanenter Öffnung des Katheterventils ist es auch für permanente Urin- oder Sekretableitung im Nachtbetrieb geeignet.

### Stand der Technik

Bei der suprapubischen oder transurethralen Harnableitung stellt die Verschlussstelle des Katheters eine der möglichen Eingangspforten für Bakterien dar. Beim Anschluss eines geschlossenen Urindrainagesystems ist diese Stelle unkritisch, da eine Diskonnektion nicht möglich ist. Beim mobilisierten Patienten findet meist eine Schiebevorrichtung oder Klemme Verwendung, die jedoch nicht immer einen dichten Verschluss gewährleisten. Die Verwendung eines einfachen Katheterstöpsels kann beim Entfernen desselben Probleme aufwerfen, da meist hohe Kräfte erforderlich sind und gegebenenfalls beim Trennungsvorgang kleine Tröpfchen oder Aerosole die Umgebung verunreinigen oder kontaminieren können.

Zur Vermeidung dieser bekannten Nachteile sind Katheterverschlussvorrichtungen bekannt geworden, die sich ohne die angeführten Nachteile relativ leicht und kontaminationsarm öffnen und schließen lassen. Die Prinzipien beruhen darauf, dass ein weicher Schlauch oder ein Membranelement entweder abgeknickt oder zusammengequetscht oder ein Dichtkörper im Bereich eines Ventilsitzes verschoben wird. In allen Fällen ist im Inneren des Konnektors eine dauerelastische Kraft wirksam, der im Bedarfsfall eine äußere Kraft entgegenwirkt.

So ist ein Katheterventil bekannt (EP 0088871), bei dem eine Stahlfeder durch axiales Verschieben einen weichen Schlauch abknickt und damit den Urinfluss unterbricht. Eine Daueröffnungsstellung für die Nachtanwendung ist nicht möglich.

Bei dem Katheterventil der Firma Uromed wird ein weicher Schlauch von einer von extern betätigten Blattfeder komprimiert und damit der Durchfluss intermittierend unterbrochen.

Ein bekanntes Katheterventil der Fa. Besola AG (Schweiz) arbeitet nach dem Prinzip einer Rollklemme, wie sie von Infusionen her bekannt sind. Das Ventil hat den großen Nachteil, dass der Schlitz, in dem die Rollklemme läuft, einen Schmutzfänger darstellt.

Den drei genannten bekannten Ventilen ist gemeinsam, dass sie zu schwer (7 bis 10 g) und zu groß sind, was den Tragekomfort für den Patienten deutlich beeinträchtigt. Der verhältnismäßig große Todraum zwischen Absperrstelle und distalem Ende des Ventils führt zu Restharn im Ventil und zum Nachtropfen von Urin. Zudem stellt diese feuchte Kammer ein ideales Milieu für das Wachstum von Bakterien dar. Die Konstruktionen sind teilweise sehr kompliziert (Verwendung von Kunststoff / Stahl), was eine teure Fertigung nach sich zieht.

Bei dem ebenfalls bekannten Ventil (EP 0144 699 B1 zum Verschluss eines Katheters, werden durch manuellen Druck extern Gelkissen, Kugeln oder andere Körper geeigneter Geometrie im inneren des Ventilkörpers verschoben, wodurch eine Öffnung bzw. ein Verschluss des Strömungskanals erreicht wird. Diese manuellen Tätigkeiten erfordern viel Fingerspitzengefühl und sind damit in vielen Fällen von älteren Patienten nicht durchführbar.

Ein magnetisch betätigtes Katheterventil (DE GM 86 23 887) arbeitet nach dem Prinzip, dass ein Verschlusskörper im Ventilgehäuse durch einen Permanentmagneten im Ventilsitz gehalten wird und das Ventil geschlossen hält. Bei Bedarf kann der Verschlusskörper durch ein externes Magnetfeld aus seiner Lage gebracht werden und den Strömungskanal freigeben.

Dieses Katheterventil hat den Nachteil, dass der Patient ständig einen Dauermagneten bei sich führen muss und die Abdichtung mittels der beweglichen Kugel nicht einwandfrei gewährleistet ist.

Die DR 532 357 gibt ein Ventil zur Entnahme von Flüssigkeiten oder Gasen an, bei dem in einem Ventilgehäuse mit einem Kegelsitz ein federbelasteter Ventilstößel das Ventil abdichtet. Über eine Ventilkappe, die axial verschiebbar auf dem Ventilgehäuse geführt wird, kann der Ventilstößel mit ausreichender Öffnungskraft gegen die Federkraft vom Kegelsitz weggedrückt werden und das Ventil wird geöffnet. Eine dauerhafte Öffnung des Ventils ist nicht möglich. Dieses Ventil weist eine Vielzahl von Spalten auf, in denen sich Bakterien ansiedeln können. Dadurch ist dieses Ventil nicht für medizinische Zwecke geeignet.

Die Erfindung hat die Aufgabe, ein leicht handhabares, einfach und preiswert herstellbares Katheterventil mit hoher Funktionssicherheit zu schaffen, dass die Nachteile des Standes der Technik vermeidet.

Die Aufgabe wird durch die Merkmale des Anspruchs 1 gelöst. Vorteilhafte Weiterbildungen der Erfindung sind in den Unteransprüchen gekennzeichnet und werden nachstehend zusammen mit der Beschreibung der bevorzugten Ausführung der Erfindung, einschließlich der Zeichnung, näher dargestellt.

Das erfindungsgemäße Katheterventil besteht aus einem zweiteiligen Gehäuse mit einer Urineintrittsöffnung und einer Urinaustrittsöffnung. In dem Gehäuse ist ein Stößel angeordnet, der ein Dichtelement aufweist. Am Stößel greift ein Federelement an, der den Stößel mit dem Dichtelement in axialer Richtung permanent gegen die Urinaustrittsöffnung drückt. Dadurch wird der Urinaustritt verhindert wird.

Nach Anspruch 2 können das Dichtelement und das Federelement auch in einem elastischen Federdichtelement kombiniert sein. Das Federdichtelement ummantelt den Stößel und liegt mit der Seite, die der Urinaustrittsöffnung gegenüber liegt, am Gehäuse an. Die kreisförmigen Stirnfläche des Federdichtelementes wird dabei gegen die Urinaustrittsöffnung gedrückt. Der Druck wird durch die Eigenspannung bzw. die Elastizität des Federdichtelements erzeugt. Dabei ist das Federdichtelement am distalsten Punkt angeordnet.

Für die optimale Handhabung des Stößels ist ein anatomisch bzw. ergonomisch ausgebildeter Schieber vorgesehen, der mit dem Stößel verbunden ist. Das Öffnen des Katheterventils kann kurzfristig mittels manueller Bewegung des Schiebers entgegen der Wirkung des Federelements oder permanent mittels eines am Gehäuse angeordneten Rastmechanismus erfolgen. Der Rastmechanismus besteht aus einem Schieber, der auf dem Gehäuse in axialer Richtung hin und her bewegt werden kann. Innerhalb des Hohlraumes im Gehäuse ist der Schieber mit dem Stößel verbunden, z.B. durch aufklipsen in einer ringförmigen Nut des Stößels.

Außen auf dem Gehäuse ist in einer am Umfang verlaufenden Nut ein drehbarer Ring angeordnet. Der Ring hat eine Aussparung, in die der Schieber bei geschlossenem Katheterventil eingreift. Wird der Schieber in axialer Richtung auf dem Gehäuse in dieser Aussparung herausgeschoben und gleichzeitig der Ring auf dem Gehäuseumfang in radialer Richtung verdreht, erfolgt eine Arretierung des Schiebers im geöffneten Zustand, so dass ein dauerhafter Urinabfluss (Nachtbetrieb) möglich ist.

Zum Ableiten des Urins kann auf einen Ansatz am Gehäuse ein Schlauch aufgesteckt werden, welcher mit einem Sammelbehälter verbunden ist.

Eine weitere erfindungsgemäße Variante einer dauerhaften Öffnung der Urinaustrittsöffnung besteht darin, dass am Schieber ein Nocken angeordnet ist, der in eine Nut in dem Gehäuse eingreift.

Die Erfindung hat eine Reihe von Vorteilen, die darin bestehen, dass das Katheterventil klein und leicht ausgeführt werden kann. Damit wird dem Patienten ein hoher Tragekomfort gewährleistet und gleichzeitig wird durch einen ausreichenden Strömungsquerschnitt eine schnelle Entleerung gewährleistet. Bedingt dadurch, dass die Urinaustrittsöffnung an der äußersten Stelle verschlossen wird, ist ein Nachtropfen ausgeschlossen. Die vorliegende Erfindung ermöglicht eine einfache Einhandbedienung, was für überwiegend geriatrische Patienten besonders vorteilhaft ist. Weiterhin wird eine einwandfreie hygienische Bedienung gesichert, so dass Infektionen der Hohlorgane und des Umfeldes des Patienten unter allen Umständen verhindert werden.

### Ausführungsbeispiel:

Anhand eines Ausführungsbeispieles und der Zeichnung soll die Erfindung näher erläutert werden. In der Zeichnung zeigen:
Fig. 1: einen axialen Schnitt durch ein erfindungsgemäßes Katheterventil;
Fig. 2: einen weiteren axialen Schnitt durch ein erfindungsgemäße Katheterventil mit einem angeschlossenen Schlauch zur Urinableitung in einen Beutel;
Fig 3: eine Ansicht A entsprechend Fig. 1 mit drehbaren Ring als Rastmechanismus;
Fig 4: eine Ansicht A entsprechend Fig 1 mit einer weiteren Möglichkeit eines Rastmechanismus.

Das Gehäuse des Katheterventils besteht aus Gründen der einfachen Fertigung aus einem ersten Gehäuseteil 2 und aus einem zweiten Gehäuseteil 1, die an einer Naht 3 miteinander verbunden sind. Das erste Gehäuseteil 2 ist an der dem Konnektor eines Urinableitungskatheters 4 zugewandten Seite konisch ausgeformt und nimmt den Urinableitungskatheter 4 auf. Dabei kann sich die Kegelfläche 5 in an sich bekannter Weise kontinuierlich oder stufig verjüngen. Der Urinableitungskatheter 4 kann auch ein Katheter zu einer beliebigen Körperhöhle sein, aus der eine Flüssigkeit oder ein Sekret abgeleitet werden soll.

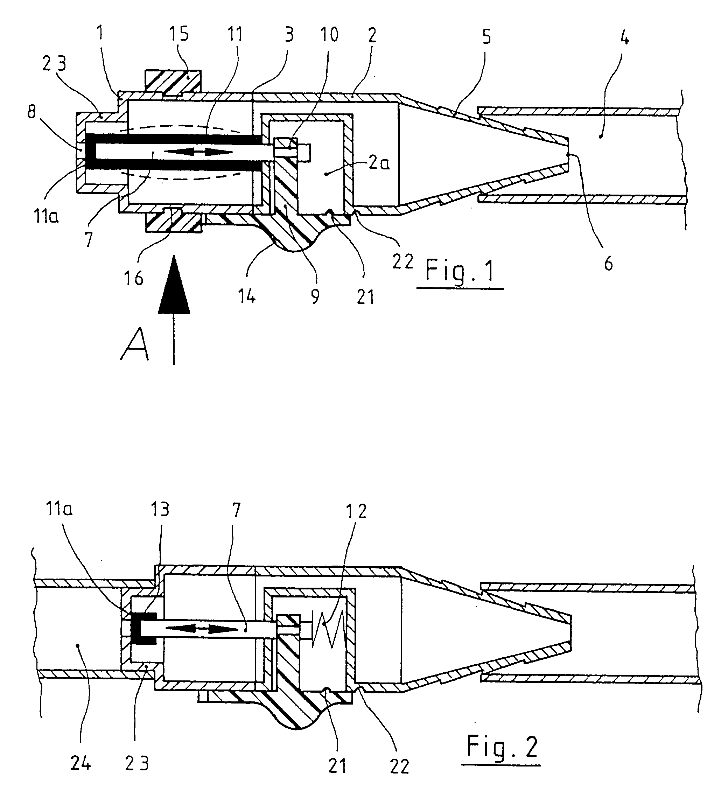

Beim Beispiel der Urinableitung tritt der Urin an einer Urineintrittsöffnung 6 in das Gehäuse des Katheterventils ein. Je nach der Stellung eines axial verschiebbaren Stößels 7, der in der Ausführung nach Figur 1 mit einem elastischem Federdichtelement umgeben ist, kann der Urin das Gehäuse des Katheterventils durch eine zentral und zugleich axial angeordnete Urinaustrittsöffnung 8 verlassen. Die axiale Bewegung des Stößels 7 erfolgt über einen Schieber 9, der mittels einer Schnappverbindung 10 auf dem Ende des Stößel 7 aufgeklipst ist, der in den Hohlraum 2a eingreift. Die Freigabe des Urinflusses erfolgt durch eine axiale Bewegung des Schieber 9 und damit des Stößels 7 nach proximal. Dabei sorgt die Geometrie des Schiebers 9 im Bereich einer Wulst 14 für ein sicheres Handling beim Öffnen und Verschließen des Katheterventils. Die Abdichtung der Urinaustrittsöffnung 8 durch den Stößel 7 erfolgt mittels Federkraft. Die Federkraft wird entweder über die Elastizität des Federdichtelements 11 (vgl. Fig. 1) oder über eine Druckfeder 12 (vgl. Fig. 2) erzeugt. Es ist auch die Anordnung einer nicht dargestellten Zugfeder möglich. Eine weitere vorteilhafte Möglichkeit besteht darin, dass an Stelle der Druckfeder 12, eine elastische Manschette angeordnet ist (in der Zeichnung nicht dargestellt). Auch die Anordnung einer Luftfeder ist technisch möglich.

Das der Urinaustrittsöffnung 8 gegenüber liegende Ende des Stößels 7 ist vorteilhafterweise plan, kegelförmig, halbkugelförmig oder in anderer, an sich bekannter Weise gut dichtender Geometrie, ausgeformt.

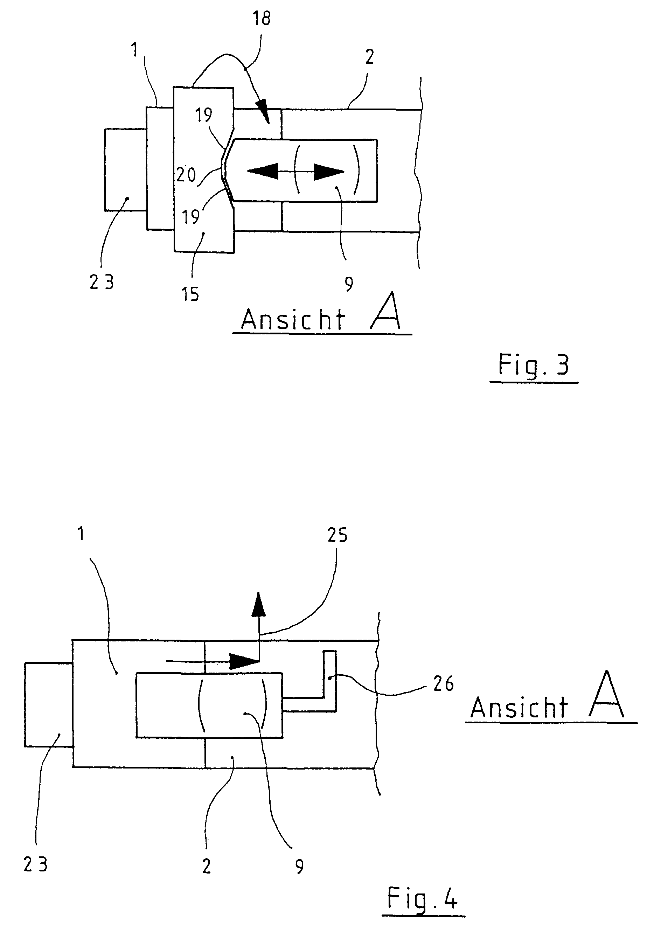

Um einen freien Urinabfluss, z.B. während der Nacht, zu gewährleisten, muss der Stößel 7 dauerhaft in Stellung proximal entgegen der Federkraft des Federdichtelements 11 bzw. der Druckfeder 12 gehalten werden. Dies wird dadurch erreicht, dass ein Ring 15, geführt in einer Aussparung 16 eine Drehbewegung 18 auf dem Gehäuse, ausführt (vgl. Fig. 1 und 3) und so den Stößel 7 in einer Stellung arretiert, die der geöffneten Urinaustrittsöffnung 8 entspricht. Die selbe Funktion kann alternativ dadurch erreicht werden, dass ein Nocken 21 an dem Schieber 9 bei der Bewegung nach proximal in eine Nut 22 einrastet (vgl. Fig. 2). Ebenso ist ein Rastmechanismus gemäß Fig. 4 möglich, wobei der Schieber 9 durch eine Zieh-Drehbewegung 25, geführt in einer L-förmigen Nut 26, arretiert wird und somit die Urinaustrittsöffnung 8 dauerhaft öffnet. Ein Verschließen erfolgt, indem der Schieber 9 in umgedrehter Bewegung der L-förmigen Nut 26 entlang geführt wird.

Zur sicheren Ableitung des Urins kann über einen Verbindungsschlauchabschnitt 24, welcher auf einen Ansatz 23 an dem Gehäuse 1 aufgeschoben wird, eine Verbindung zu einem Sammelbehältnis hergestellt werden (Fig. 2).

Aufstellung der verwendeten Bezugszeichen:
- 1: zweites Gehäuseteil
- 2: erstes Gehäuseteil
- 2a: Hohlraum
- 3: Naht
- 4: Urinableitungskatheter
- 5: Kegelfläche
- 6: Urineintrittsöffnung
- 7: Stößel
- 8: Urinaustrittsöffnung
- 9: Schieber
- 10: Schnappverbindung
- 11: Federdichtelement
- 11a: Stirnfläche
- 12: Druckfeder
- 13: elastisches Bauteil
- 14: Wulst
- 15: Ring
- 16: Aussparung
- 17 18: Drehbewegung
- 19: Schrägen
- 20: V-förmige Aussparung
- 21: Nocken
- 22: Nut
- 23: Ansatz
- 24: Verbindungsschlauchabschnitt
- 25: Zieh-Drehbewegung
- 26: L-förmige Nut

## Patentansprüche

1. Katheterventil bestehend aus einem zweiteiligen Gehäuse, welches ein erstes Gehäuseteil (2) mit einer Urin-eintrittsöffnung (6) und ein zweites Gehäuseteil (1) mit einer Urinaustrittsöffnung (8) sowie einen nach außen offenen Hohlraum (2a) aufweist, und einem im Gehäuse axial zur Urinaustrittsöffnung (8) verschiebbar gelagerten Stößel (7) mit einem Dichtelement, welches unter der Wirkung eines Federelementes die Urinaustrittsöffnung (8) abdichtet, wobei das andere Ende des Stößels (7) in den Hohlraum (2a) eingreift und an einen außen am Gehäuse geführten Schieber (9) angelenkt ist, mit dem der Stößel geführt wird.

2. Katheterventil nach Anspruch 1, **dadurch gekennzeichnet, dass** als Dichtelement und Pederelement ein elastisches Federdichtelement (11) vorhanden ist, welches den Stößel (7) ummantelt und mit der Seite, die der Urinaustrittsöffnung (8) gegenüber liegt, mit der Wirkung innerer Druckspannung am Gehäuse anliegt.

3. Katheterventil nach Anspruch 1, **dadurch gekennzeichnet, dass** als Federelement eine Druckfeder (12) vorhanden ist, die innerhalb des Hohlraumes (2a) gelagert ist und axial gegen den Stößel (7) wirkt.

4. Katheterventil nach einem der Ansprüche 1 bis 3, **dadurch gekennzeichnet, dass** ein Rastmechanismus vorhanden ist, bestehend aus einem Ring (15), der in einer Aussparung (16) am Gehäuse drehbar gehaltert ist und eine V-förmigen Aussparung (20) mit Schrägen (19) aufweist, in die der Schieber (9) eingreift, wobei die Schrägen (19) und der Schieber (9) derart ausgebildet sind, dass der Schieber (9) bei Drehung des Ringes (15) aus der V-förmigen Aussparung (20) herausdrückt und die Urinaustrittsöffnung (6) geöffnet wird.

5. Katheterventil nach einem der Ansprüche 1 bis 3, **dadurch gekennzeichnet, dass** am Schieber (9) ein Nocken (21) vorhanden ist, der beim Öffnen der Urinaustrittsöffnung (6) in eine Nut (22) am Gehäuse derart einrasten kann, dass der Schieber (9) gegen die Wirkung des Federelementes gehalten wird.

6. Katheterventil nach einem der Ansprüche 1 bis 5, **dadurch gekennzeichnet, dass** der Schieber (9) zur optimalen Handhabung des Katheterventils eine ergonomisch ausgebildete Angriffsfläche aufweist.

## Claims

1. Catheter valve consisting of a two-component housing which has a first housing component (2) with a urine inlet opening (6) and a second housing component (1) with a urine outlet opening (8), as well as a hollow cavity (2a) open to the outside, and of a shaft (7) which is mounted in the housing in such a way that it can be displaced axially with respect to the urine outlet opening (8) and which has a sealing element which seals off the urine outlet opening (8) under the action of a spring element, the other end of the shaft (7) engaging in the hollow cavity (2a) and being articulated on a slide (9) which is guided on the outside of the housing and with which the shaft is guided.

2. Catheter valve according to Claim 1, **characterized in that** sealing element and spring element are provided in the form of an elastic resilient sealing element (11) which encloses the shaft (7) and, via the side lying opposite the urine outlet opening (8), bears on the housing under the effect of internal compressive stress.

3. Catheter valve according to Claim 1, **characterized in that** the spring element provided is a compression spring (12) which is mounted inside the hollow cavity (2a) and acts axially against the shaft (7).

4. Catheter valve according to one of Claims 1 to 3, **characterized in that** a locking mechanism is provided, consisting of a ring (15) which is mounted rotatably in a recess (16) on the housing and has a V-shaped recess (20) with bevels (19) in which the slide (9) engages, the bevels (19) and the slide (9) being designed in such a way that the slide (9), upon rotation of the ring (15), is pressed out of the V-shaped recess (20) and the urine outlet opening (6) is opened.

5. Catheter valve according to one of Claims 1 to 3, **characterized in that** the slide (9) is provided with a cam (21) which, when the urine outlet opening (6) is opened, can catch in a groove (22) on the housing in such a way that the slide (9) is held counter to the action of the spring element.

6. Catheter valve according to one of Claims 1 to 5, **characterized in that**, for optimum handling of the catheter valve, the slide (9) has an ergonomically designed grip surface.

## Revendications

1. Soupape de cathéter se composant d'un logement en deux parties, qui présente une première partie de logement (2) comprenant une ouverture d'entrée pour l'urine (6) et une deuxième partie de logement (1) comprenant une ouverture de sortie pour l'urine (8) ainsi qu'un espace creux ouvert vers l'extérieur (2a), et d'une tige-poussoir (7) montée déplaçable dans le logement axialement par rapport à l'ouverture de sortie pour l'urine (8), comprenant un élément de joint qui ferme de manière étanche l'ouverture de sortie pour l'urine (8) sous l'action d'un élément de ressort, l'autre extrémité de la tige-poussoir (7) s'engageant dans l'espace creux (2a) et étant articulée à un tiroir (9) guidé à l'extérieur sur le logement, avec lequel la tige-poussoir est guidée.

2. Soupape de cathéter selon la revendication 1, **caractérisée en ce qu'**un élément de joint à ressort élastique (11) est prévu en tant qu'élément de joint et élément de ressort, lequel enveloppe la tige-poussoir (7) et repose, avec son côté en regard de l'ouverture de sortie pour l'urine (8), contre le logement sous l'action d'une tension de compression interne.

3. Soupape de cathéter selon la revendication 1, **caractérisée en ce qu'**un ressort de compression (12) est prévu en tant qu'élément de ressort, lequel est monté à l'intérieur de l'espace creux (2a) et agit axialement contre la tige-poussoir (7) .

4. Soupape de cathéter selon l'une quelconque des revendications 1 à 3, **caractérisée en ce qu'**il est prévu un mécanisme d'encliquetage, se composant d'une bague (15) qui est montée rotative dans un évidement (16) sur le logement, et présente un évidement en forme de V (20) avec des biseaux (19), dans lequel le tiroir (9) s'engage, les biseaux (19) et le tiroir (9) étant configurés de telle sorte que le tiroir (9) sorte de l'évidement en forme de V (20) lors de la rotation de la bague (15) et que l'ouverture de sortie pour l'urine (8) s'ouvre.

5. Soupape de cathéter selon l'une quelconque des revendications 1 à 3, **caractérisée en ce qu'**il est prévu sur le tiroir (9) une came (21) qui peut s'encliqueter dans une rainure (22) sur le logement lors de l'ouverture de l'ouverture de sortie pour l'urine (8) de telle sorte que le tiroir (9) soit maintenu à l'encontre de l'action de l'élément de ressort.

6. Soupape de cathéter selon l'une quelconque des revendications 1 à 5, **caractérisée en ce que** le tiroir (9) présente une surface de prise de forme ergonomique pour une manipulation optimale de la soupape de cathéter.
